# EUROPÄISCHE PATENTSCHRIFT

(11) **EP 1 284 962 B1**
(45) Veröffentlichungstag und Bekanntmachung des Hinweises auf die Patenterteilung: **16.12.2009**
(21) Anmeldenummer: 01960244.0
(22) Anmeldetag: 25.05.2001
(51) Int. Cl.: C07D 207/267

(54) **VERFAHREN ZUR GEWINNUNG VON REINEM N-VINYLPYRROLIDON**
METHOD FOR OBTAINING PURE N-VINYL PYRROLIDONE
PROCEDE POUR OBTENIR DU N-VINYLPYRROLIDONE PUR

(30) Priorität: 26.05.2000 DE 10026233
(43) Veröffentlichungstag der Anmeldung: 26.02.2003
(73) Patentinhaber: BASF SE, 67056 Ludwigshafen (DE)
(72) Erfinder: ECK, Bernd, 68519 Viernheim (DE); HEILEK, Jörg, 69245 Bammental (DE); SCHMIDT-RADDE, Martin, 67259 Beindersheim (DE); HELFERT, Herbert, 67227 Frankenthal (DE)
(74) Vertreter: Pohl, Michael Friedrich
(86) Internationale Anmeldenummer: PCT/EP2001/006006
(87) Internationale Veröffentlichungsnummer: WO 2001/090066

(56) Entgegenhaltungen:
- EP-A- 0 767 169
- DE-A- 3 928 982
- US-A- 4 873 336
- US-A- 5 508 396

## Beschreibung

Die vorliegende Erfindung betrifft ein Verfahren zur Gewinnung von reinem N-Vinylpyrrolidon aus N-Vinylpyrrolidon-haltigen Rohprodukten.

Homo- und Copolymere des N-Vinylpyrrolidons finden in vielen Bereichen Anwendung. Häufig finden sie als Monomere zur Herstellung von Polymeren für kosmetische Produkte, für den Pharmabereich sowie für die Lebensmitteltechnik Verwendung. Für die letztgenannten Bereiche werden hohe Anforderungen an die Reinheit des N-Vinylpyrrolidons gesetzt.

In industriellem Maßstab wird N-Vinylpyrrolidon durch Vinylierung von 2-Pyrrolidon mit Acetylen unter Druck in Gegenwart basischer Katalysatoren, wie Hydroxiden und Alkoholaten, hergestellt. Das nach Destillation erhaltene Produkt enthält das Wertprodukt in der Regel in einer Menge von 98 bis 99,9 %, wobei die Hauptverunreinigung 2-Pyrrolidon ist. Daneben enthält das Produkt in geringer Menge noch Stickstoff- und Vinyletherverbindungen, die zum Teil bei der Polymerisation zu Produktverfärbungen und zur Geruchsbildung führen.

Für eine Vielzahl von Anwendungen wird ein N-Vinylpyrrolidon benötigt, das weniger als 0,1 Gew.-% Verunreinigungen enthält. Grundsätzlich kann ein solcher Reinheitsgrad durch eine fraktionierte Destillation erreicht werden. Eine Abtrennung der Verbindungen, die für die bei der Polymerisation beobachtete Produktverfärbung verantwortlich sind, ist auf diesem Weg nur schlecht möglich. Häufig kombiniert man daher die Destillation mit einer extraktiven Aufarbeitung. In der DE 37 36 603 wird beispielsweise die Entfernung derartiger Verunreinigungen mit Hilfe von Ionenaustauschern beschrieben.

Aus der WO 94/18166 ist die Reinigung von N-Vinylpyrrolidon durch eine mehrstufige, fraktionierte Kristallisation bekannt. Bei diesem Verfahren wird eine unterkühlte Schmelze des N-Vinylpyrrolidons kristallisiert, die Mutterlauge abgetrennt und das Kristallisat wenigstens einer weiteren Kristallisationsstufe unterworfen. Die Gewinnung des Wertprodukts aus der Mutterlauge erfolgt ebenfalls durch mehrstufige fraktionierte Kristallisation. Das aus der WO 94/18166 bekannte Verfahren beruht auf dem Gegenstromprinzip, bei dem in jeder Kristallisationsstufe das Kristallisat vom Kristallisationsrückstand (Mutterlauge) abgetrennt wird, das Kristallisat der nächsthöheren Kristallisationsstufe zugeführt und der Kristallisatrückstand (Mutterlauge) der nächstunteren Kristallisationsstufe zugeführt wird. Der apparative Aufwand für ein derartiges Kristallisationsverfahren ist naturgemäß hoch. Zudem ist das Verfahren aufgrund der Vielzahl der Kristallisationsstufen zeitaufwendig.

Die EP-A 767 169 beschreibt ebenfalls ein Kristallisationsverfahren zur Reinigung von N-Vinylpyrrolidon, das sich von dem aus der WO 94/18166 bekannten Verfahren dadurch unterscheidet, dass man anstelle durch Unterkühlung der Schmelze die Kristallisation des N-Vinylpyrrolidons bei der Kristallisationstemperatur mittels Impfkristallen einleitet. Auch hier erfolgt die Aufarbeitung der Mutterlauge der ersten Kristallisationsstufe durch eine oder mehrere aufeinanderfolgende Kristallisationsstufen. Auch wenn aufgrund der höheren Effizienz weniger Kristallisationsstufen erforderlich sind als bei dem Verfahren der WO 94/18166, ist der apparative Aufwand auch für dieses Verfahren recht hoch.

Der vorliegenden Erfindung liegt die Aufgabe zugrunde, ein Verfahren zur Gewinnung von reinem N-Vinylpyrrolidon aus N-Vinylpyrrolidon-haltigen Rohprodukten bereitzustellen, das mit einem geringen apparativen Aufwand betrieben werden kann und N-Vinylpyrrolidon hoher Reinheit liefert. Außerdem soll sich das Verfahren vorteilhaft in ein kontinuierliches Verfahren zur Herstellung von N-Vinylpyrrolidon einbinden lassen.

Diese Aufgabe wird gelöst durch ein Verfahren zur Gewinnung von reinem N-Vinylpyrrolidon aus N-Vinylpyrrolidon-haltigen Rohprodukten, umfassend ein ein- oder mehrstufiges Kristallisationsverfahren, das dadurch gekennzeichnet ist, dass man die Mutterlauge der ersten Kristallisationsstufe einer destillativen Reinigung zuführt.

Das erfindungsgemäße Verfahren zur Aufreinigung von N-Vinylpyrrolidon kann grundsätzlich sowohl in ein diskontinuierliches Verfahren als auch in ein kontinuierliches Verfahren zur Herstellung von N-Vinylpyrrolidon eingebunden werden. Besonders vorteilhaft ist die Einbindung des erfindungsgemäßen Aufreinigungsverfahrens in ein kontinuierliches Herstellungsverfahren von N-Vinylpyrrolidon.

Im erfindungsgemäßen Verfahren gewinnt man das reine N-Vinylpyrrolidon aus dem Rohprodukt durch Kristallisation des Rohprodukts, die man ein- oder mehrstufig durchführen kann. Selbstverständlich führt man die Kristallisation so durch, dass eine Trennung in eine feste, d. h. kristalline, mit N-Vinylpyrrolidon angereicherte Phase, und eine flüssige, von N-Vinylpyrrolidon abgereicherte Phase (Mutterlauge) erhalten wird.

Erfindungsgemäß wird die bei der Kristallisation anfallende Mutterlauge der ersten Kristallisationsstufe, d. h. der Kristallisationsstufe, in die man das N-Vinylpyrrolidon-haltige Rohprodukt einspeist, keiner weiteren kristallisierenden Aufarbeitung unterworfen. Vielmehr führt man zur Aufarbeitung der Mutterlauge diese einer destillativen Reinigung, vorzugsweise einer kombinierten extraktiven und destillativen Reinigung zu.

Unter einer destillativen Reinigung versteht man hier eine ein- oder mehrstufige fraktionierte Destillation, bei der aus der Mutterlauge sogenannte Leichtsieder, d. h. Verbindungen mit einem höheren Dampfdruck als N-Vinylpyrrolidon, und sogenannte Schwersieder, d. h. Verbindungen mit einem geringeren Dampfdruck als N-Vinylpyrrolidon, insbesondere 2-Pyrrolidon selber, vom Wertprodukt abgetrennt werden. Vorzugsweise führt man die destillative Reinigung als Vakuumdestillation durch. Die Destillationstemperaturen liegen in der Regel im Bereich von 50 bis 150 °C und insbesondere im Bereich von 80 bis 120 °C bei einem Druck von 1 bis 100 Torr und insbesondere von 5 bis 20 Torr. Die hierbei anfallenden Leichtsieder, insbesondere 2-Pyrrolidon-haltige Fraktionen, kann man über die Rohdestillation oder unmittelbar in den eigentlichen Herstellungsprozess zurückführen. Vorzugsweise kombiniert man die destillative Reinigung mit einer extraktiven Reinigung.

In einer besonders bevorzugten Ausgestaltung des erfindungsgemäßen Verfahrens teilt man das zu reinigende Rohprodukt in zwei Teilströme T1 und T2 auf. Der Teilstrom T1 wird der Kristallisation zugeführt. Der Teilstrom T2 wird einer destillativen Aufarbeitung, wie oben beschrieben, unterworfen.

Die bei der kristallisierenden Aufarbeitung anfallende Mutterlauge kann dem Teilstrom T2 zugeführt werden. Über den Teilstrom T2 wird die Mutterlauge dann der destillativen Aufarbeitung zugeführt. Diese Vorgehensweise hat zum einen den Vorteil, dass man je nach Bedarf und Anforderungen an das N-Vinylpyrrolidon gezielt Produkte mit unterschiedlichen Qualitäten herstellen kann. Zum anderen ermöglicht diese Vorgehensweise die einfache Einbindung eines Verfahrens zur Herstellung eines hochreinen N-Vinylpyrrolidons durch Kristallisation in ein bestehendes, kontinuierliches Herstellungsverfahren, bei dem die Reinigung über destillative Schritte erfolgt. Weiterhin ermöglicht dieses Verfahren die einfache Einbindung der rückzuführenden Mutterlauge aus dem Kristallisationsprozess in ein bestehendes destillatives Aufarbeitungskonzept. Eine aufwendige Aufarbeitung der Mutterlauge entfällt. Außerdem kann die Mutterlauge teilweise dem Strom T1 zurückgeführt werden.

Das im erfindungsgemäßen Verfahren der Kristallisation zuzuführende N-Vinylpyrrolidon-haltige Rohprodukt unterliegt an sich keiner Beschränkung. Bevorzugt enthält das N-Vinylpyrrolidon-haltige Rohprodukt nicht mehr als 20 Gew.-%, vorzugsweise nicht mehr als 10 Gew.-% und insbesondere 0,3 bis 2 Gew.-% 2-Pyrrolidon und nicht mehr als 1 Gew.-% und insbesondere nicht mehr als 0,1 Gew.-% leichtsiedende und/oder farbgebende Verunreinigungen. Derartige Rohprodukte werden in der Regel durch destillative Aufarbeitung (Rohdestillation) des bei der Herstellung des N-Vinylpyrrolidon anfallenden Produktstroms gewonnen. Vorzugsweise führt man die Rohdestillation in Form einer Vakuumdestillation durch, bei der das Wertprodukt N-Vinylpyrrolidon über Kopf abdestilliert und ein Großteil des 2-Pyrrolidons als Sumpfprodukt abgezogen wird. Letzteres führt man in der Regel in den Herstellungsprozess zurück.

Die eigentliche Kristallisation des N-Vinylpyrrolidon-haltigen Rohprodukts kann in Analogie zu bekannten Kristallisationsverfahren erfolgen. Geeignete Kristallisationsverfahren sind beispielsweise aus der US 5,329,021, der DE-A 26 06 364, der DE-A 17 69 123 und der EP-A 475 893 bekannt. Zur Durchführung der Kristallisation überführt man das zu reinigende Rohprodukt in der Regel in einen Kristallisator und kristallisiert unter Kühlen des Rohprodukts einen Teil des N-Vinylpyrrolidons aus. Die dabei anfallende N-Vinylpyrrolidon-haltige Mutterlauge wird abgetrennt und den in Anspruch 1 angegebenen Maßnahmen unterworfen. Vorzugsweise führt man die Kristallisation so weit, dass wenigstens 5 Gew.-% und vorzugsweise wenigstens 20 Gew.-% des in dem der Kristallisation zugeführten Rohprodukt enthaltenen N-Vinylpyrrolidons auskristallisiert sind. In der Regel wird man nicht mehr als 95 Gew.-%, insbesondere nicht mehr als 80 Gew.-% des in dem Rohprodukt enthaltenen N-Vinylpyrrolidon auskristallisieren, um eine hinreichende Reinigungswirkung zu erzielen.

Der im erfindungsgemäßen Verfahren eingesetzte Kristallisator unterliegt an sich keiner Einschränkung. Als besonders geeignet haben sich Kristallisatoren erwiesen, deren Funktion auf der Bildung von Kristallen auf gekühlten Flächen beruht. Derartige Kristallisationsverfahren werden auch als Schichtkristallisation bezeichnet. Geeignete Apparate sind in der DE-OS 17 69 123, der DE-OS 26 06 364, der EP-A 218 545, der EP-A 323 377, der CH 645278, der FR 2668946 und der US 3,597,164 beschrieben.

Zur Schichtkristallisation wird das N-Vinylpyrrolidon enthaltende Rohprodukt mit den gekühlten Flächen des Wärmetauschers in Kontakt gebracht. Dabei kühlt man die Wärmetauscherflächen des Kristallisators vorzugsweise auf Temperaturen, die bis 40 K unterhalb der Schmelztemperatur des N-Vinylpyrrolidons in dem Rohprodukt liegen können. Bei Erreichen des gewünschten Kristallisationsgrades wird der Abkühlvorgang beendet und die flüssige Mutterlauge abgeführt, z. B. durch Abpumpen oder Abfließen. Die Isolierung des gereinigten, kristallisierten-N-Vinylpyrrolidons erfolgt in der Regel durch Erwärmen der Wärmetauscherflächen auf eine Temperatur oberhalb der Schmelztemperatur des N-Vinylpyrrolidons, wobei das gereinigte N-Vinylpyrrolidon als Schmelze anfällt und als solche isoliert wird. Gegebenenfalls führt man vor der Isolierung des gereinigten N-Vinylpyrrolidons noch weitere Reinigungsschritte durch.

Als zusätzlichen Reinigungsschritt kann man beispielsweise ein Schwitzen der auf den Wärmetauscherflächen abgeschiedenen Kristallschicht durchführen. Hierbei wird die Temperatur der Kristallschicht etwas angehoben, wobei bevorzugt die höher verunreinigten Bereiche der Kristallschicht abschmelzen und so eine zusätzliche Reinigungswirkung erzielt wird. Das Schwitzprodukt wird dann der Mutterlauge zugeführt und mit dieser weiter verarbeitet. Auch kann man die Kristallschicht mit einer Reinigungsflüssigkeit, beispielsweise einer Schmelze von aufgereinigtem N-Vinylpyrrolidon, behandeln.

Die zur Schichtkristallisation erforderliche Temperatur des Rohprodukts im Kristallisator hängt von seiner Zusammensetzung ab. Die Obergrenze ist naturgemäß die Temperatur, bei der sich das bereits kristallisierte N-Vinylpyrrolidon mit dem in der Mutterlauge enthaltenen N-Vinylpyrrolidon im Gleichgewicht befindet (Gleichgewichtstemperatur). Je nach Zusammensetzung des Rohprodukts liegt die Gleichgewichtstemperatur im Bereich von +14,4 bis -6 °C. Selbstverständlich kann man auch niedrigere Temperaturen zur Kristallisation anwenden. Vorzugsweise vermeidet man jedoch stark unterkühlte Schmelzen, so dass die zur Kristallisation angewendete Temperatur des Rohprodukts vorzugsweise nicht mehr als 5 °C und insbesondere nicht mehr als 2 °C unterhalb der Gleichgewichtstemperatur liegt. Typischerweise liegt die Temperatur des Rohprodukts während der Schichtkristallisation im Bereich von -10 bis +14,4 °C und besonders bevorzugt im Bereich von -5 bis +14 °C.

Es hat sich als vorteilhaft erwiesen, wenn man die Schichtkristallisation in Gegenwart von Impfkristallen durchführt. Die Anwendung von Impfkristallen bei der Kristallisation des N-Vinylpyrrolidons ist grundsätzlich aus der EP-A 767 169 bekannt, so dass diesbezüglich in vollem Umfang auf diese Schrift Bezug genommen wird. Dabei arbeitet man vorzugsweise so, dass man vor der Kristallisation diejenigen Flächen des Kristallisators, von denen aus während der Kristallisation Kristalle wachsen, mit einer Impfschicht aus N-Vinylpyrrolidon belegt. Die Impfkristalle können sowohl aus dem zu reinigenden Rohprodukt als auch aus einer Schmelze von gereinigtem N-Vinylpyrrolidon gewonnen werden. Beispielsweise kann man Impfkristalle auf den Flächen des Kristallisators, an denen das Kristallwachstum stattfinden soll, erzeugen, indem man einen N-Vinylpyrrolidon-haltigen Schmelzfilm auf diesen Flächen erzeugt und diesen anfriert, beispielsweise durch Kühlen auf eine Temperatur unterhalb der Schmelztemperatur. Vorzugsweise erfolgt die Erzeugung der Impfkristalle durch Aufbringen eines Films aus einer Suspension von N-Vinylpyrrolidon-Kristallen in einer N-Vinylpyrrolidon-Schmelze und anschließendes Anfrieren dieses Films. Das Anfrieren erfolgt hier vorzugsweise bei einer Temperatur im Bereich der Gleichgewichtstemperatur.

Eine derartige Suspension kann man erzeugen, indem man aus dem Rohprodukt oder einer Schmelze des gereinigten N-Vinylpyrrolidons eine geringe Menge an Kristallen durch Unterkühlung ausfriert. Beispielsweise kann man in Kratzkühlern oder Rührkesseln mit wandgängigen Rührern durch indirekte Kühlung aus einer N-Vinylpyrrolidon-haltigen Schmelze (Rohprodukt oder Schmelze gereinigten Vinylpyrrolidons) Kristalle ausfrieren, die mit Hilfe von Schabelementen an den gekühlten Wänden in der Schmelze suspendiert werden. Man kann die Impfkristalle auch direkt in der Schmelze erzeugen, indem man die Schmelze entweder über den Kristallisator oder über im Kristallisator eingebaute, kühlbare Elemente (beispielsweise Kühlfinger oder Kühlstrecken) auf eine Temperatur unterhalb der Schmelztemperatur abkühlt. Vorzugsweise erzeugt man Impfkristalle in einer Menge von 0,1 bis 700 g/kg Schmelze und insbesondere im Bereich von 1 bis 300 g/kg Schmelze.

In einer bevorzugten Ausgestaltung der Schichtkristallisation bringt man die Suspension auf die Kristallisatorflächen auf, indem man den Kristallisator mit der Suspension füllt und anschließend entleert. Nach Entleerung verbleibt eine Suspensionsschicht auf den Kristallisatorflächen, die dann, vorzugsweise im Bereich ihrer Gleichgewichtstemperatur, angefroren wird.

Die Kristallisation an Kühlflächen kann als dynamisches oder statisches Verfahren durchgeführt werden. Vorzugsweise finden dynamische Verfahren Anwendung. Statische Verfahren sind beispielsweise in der US 3,597,164, der EP 323 377 und der FR 2668946 beschrieben, auf die hiermit Bezug genommen wird. Bei dem statischen Verfahren findet ein Stoffaustausch in der flüssigen Phase nur durch freie Konvektion statt (ruhende Schmelze).

Bei den dynamischen Verfahren der Kristallisation wird das zu kristallisierende Rohprodukt in einer strömenden Bewegung gehalten. Dies kann durch eine erzwungene Strömung in voll durchströmten Wärmeüberträgern erfolgen, wie z. B. in der DE 26 06 364 beschrieben, oder durch die Aufgabe eines Rieselfilms auf eine gekühlte Wand, wie es beispielsweise in der DE-AS 1 769 123 und der EP-A 218 545 beschrieben wird, oder mittels bewegter Kühlflächen wie Kühlwalzen oder Kühlbänder. Vorzugsweise erfolgt die dynamische Schichtkristallisation in voll durchströmten Wärmeüberträgern, beispielsweise in von außen gekühlten Rohren oder Rohrbündeln.

Bei den dynamischen Schichtkristallisationsverfahren, insbesondere solchen, die in voll durchströmten Wärmeaustauschern durchgeführt werden, geht man in der Regel so vor, dass man das N-Vinylpyrrolidon-haltige Rohprodukt, gegebenenfalls nach Aufbringen der Impfkristallschicht auf den Wärmetauscherflächen des Kristallisators, mit den gekühlten Wärmetauscherflächen in Kontakt bringt, beispielsweise indem man das Rohprodukt durch die gekühlten Rohre des Kristallisators strömen lässt. Hierbei kristallisiert das N-Vinylpyrrolidon aus dem Rohprodukt zumindest teilweise aus. In der Regel wird dieser Vorgang abgebrochen, wenn aufgrund der auskristallisierten Menge an N-Vinylpyrrolidon ein ausreichendes Durchströmen der Schmelze durch den Wärmetauscher gerade noch möglich ist. Hierzu entfernt man die flüssige Phase (Mutterlauge) und isoliert danach das kristallisierte N-Vinylpyrrolidon in der oben beschriebenen Weise, z. B. indem man gegebenenfalls nach einem weiteren Reinigungsschritt die Wärmetauscherflächen auf eine Temperatur oberhalb der Schmelztemperatur des N-Vinylpyrrolidons erwärmt. Dieser Vorgang kann mehrmals wiederholt werden, bis die gewünschte Menge an N-Vinylpyrrolidon aus dem Rohprodukt auskristallisiert ist.

Die Kristallisation kann als Alternative zur Schichtkristallisation auch als Suspensionskristallisation durchgeführt werden. Bei der Suspensionskristallisation werden in dem N-Vinylpyrrolidon enthaltenden, flüssigen Rohprodukt durch Wärmeabfuhr in der Masse Einzelkristalle gebildet. Die dabei entstehende Kristallsuspension wird während des Suspensionskristallisationsverfahrens bewegt, wozu insbesondere ein Umpumpen oder Rühren geeignet ist. Ein Anhaften von Kristallen an Wärmetauscherflächen ist hierbei nicht erforderlich und sogar unerwünscht. Die Suspensionskristallisation zählt naturgemäß zu den dynamischen Kristallisationsverfahren, da das Rohprodukt während des Kristallisierens bewegt wird. Hinsichtlich der zur Kristallisation des N-Vinylpyrrolidons erforderlichen Temperaturen des Rohprodukts gilt das oben Gesagte.

Bei der Suspensionskristallisation erfolgt die Abfuhr der Wärme in der Regel durch indirekte Kühlung, beispielsweise über Kratzkühler, die mit einem Rührkessel oder einem Behälter ohne Rührwerk verbunden sind. Der Umlauf der Kristallsuspension wird hierbei durch eine Pumpe gewährleistet. Daneben besteht auch die Möglichkeit, die Wärme über Wände des Rührkessels mit wandgängigen Rührern abzuführen. Zur Wärmeabfuhr ist auch die Verwendung von Kühlscheibenkristallisatoren geeignet, wie sie beispielsweise von der Fa. GMF (Gouda in Holland) hergestellt werden. Selbstverständlich kann man die Wärme auch durch Kühlung über herkömmliche Wärmeüberträger (bevorzugt Rohrbündel- oder Plattenwärmeüberträger) abführen. Hierbei kommt es allerdings im Unterschied zu den vorgenannten Maßnahmen zur Wärmeabfuhr zur Bildung von Kristallschichten auf den wärmeübertragenden Flächen. Wird im Betrieb ein Zustand erreicht, bei dem der Wärmedurchgangswiderstand durch Verkrustung einen zu hohen Wert annimmt, erfolgt die Umschaltung auf einen zweiten Wärmeüberträger. Während der Betriebszeit des zweiten Wärmeüberträgers wird der erste Wärmeüberträger dann regeneriert, beispielsweise durch Abschmelzen der Kristallschicht. Wird dann im zweiten Wärmeüberträger ein zu hoher Wärmedurchgangswiderstand erreicht, schaltet man auf den ersten Wärmeüberträger zurück. Diese Maßnahme kann auch mit mehr als zwei Wärmeüberträgern im Wechsel betrieben werden. Eine Abfuhr der Wärme kann auch durch konventionelle Teilverdampfung des Rohprodukts im Vakuum erfolgen.

Die Trennung des bei der Suspensionskristallisation anfallenden, mit N-Vinylpyrrolidon angereicherten Kristallisats von der abgereicherten Mutterlauge gelingt nach den hierzu bekannten Verfahren der Fest-Flüssig-Trennung, beispielsweise durch Filtration, Sedimentieren und/oder Zentrifugieren. Bei einem ruhenden Kristallisat kann man die Mutterlauge auch entfernen, indem man sie ablaufen lässt. Beim Filtrieren, Sedimentieren oder Zentrifugieren führt man vorzugsweise zuvor eine Voreindickung der Suspension durch, beispielsweise mittels Hydrozyklone. Zum Zentrifugieren eignen sich alle bekannten Zentrifugen, die diskontinuierlich oder kontinuierlich arbeiten. Besonders vorteilhaft setzt man Schubzentrifugen ein, die ein- oder mehrstufig betrieben werden können. Daneben eignen sich auch Schneckensiebzentrifugen oder Schneckenaustragszentrifugen (Dekanter). Die Filtration erfolgt in der Regel mittels Filternutschen, die kontinuierlich oder diskontinuierlich, mit oder ohne Rührwerk, oder mittels Bandfilter betrieben werden. Das Filtrieren kann unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. Vorzugsweise wird man nach dem Abtrennen der Kristalle von der Mutterlauge ein ein- oder mehrstufiges Waschen und/oder Schwitzen der Kristalle oder des Kristallkuchens durchführen. Als Waschflüssigkeit wird man vorzugsweise flüssiges N-Vinylpyrrolidon einsetzen, dessen Reinheit oberhalb derer der Mutterlauge liegt. Das Waschen kann in den hierfür üblichen Apparaten erfolgen, beispielsweise in Zentrifugen oder in Filternutschen oder Bandfiltern. Das Waschen kann ein- oder mehrstufig durchgeführt werden, wobei die Waschflüssigkeit vorzugsweise im Gegenstrom zum Kristallkuchen geführt wird. Bei einer mehrstufigen Kristallisation wird in besonders geeigneter Weise als Waschflüssigkeit für das Kristallisat einer jeweiligen Kristallisationsstufe, der Zulauf zu derselben Kristallisationsstufe eingesetzt. Bevorzugt liegt das Massenverhältnis von Waschflüssigkeit zu Kristallisat im Bereich von 0,1 bis 1, besonders bevorzugt im Bereich von 0,2 bis 0,6 kg Waschflüssigkeit zu kg Kristallisat.

Besonders bevorzugt erfolgt eine Aufreinigung des Kristallisats bei der Suspensionskristallisation durch Durchführung des oben beschriebenen Waschens, insbesondere auf Zentrifugen oder Bandfiltern. Selbstverständlich ist auch eine kombinierte Durchführung von Wasch- und Schwitzvorgängen in einem Apparat möglich.

Zur Aufreinigung des bei der Suspensionskristallisation anfallenden Kristallisats werden vorzugsweise Waschkolonnen eingesetzt, in denen das Kristallisat in der Regel nach einer Voreindickung z. B. durch Filtration oder Sedimentation, im Gegenstrom zu einer Waschflüssigkeit geführt wird, wobei man kontinuierlich oder diskontinuierlich arbeiten kann. Als Waschflüssigkeit wird bevorzugt eine Schmelze des bereits gereinigten Kristallisats verwendet. Der Transport der Kristalle entgegen der Strömungsrichtung kann in üblicher Weise, z. B. mittels Schwerkraft, vorzugsweise durch mechanische Förderung oder durch hydraulische Kräfte (z. B. Strömungsdruckverluste beim Durchströmen des Kristallhaufwerks) erfolgen.

Alle der vorgenannten Kristallisationsverfahren können kontinuierlich oder diskontinuierlich betrieben werden.

Die bevorzugte dynamische Schichtkristallisation erfolgt vorzugsweise diskontinuierlich, insbesondere wenn sie in voll durchströmten Wärmeüberträgern, wie oben beschrieben, erfolgt. Eine Einbindung in ein kontinuierliches Herstellungsverfahren ist jedoch ohne Weiteres möglich, beispielsweise mit Hilfe von Behältern zur Zwischenspeicherung des Rohprodukts oder durch Verändern des Verhältnisses der Teilströme T1 und T2.

Das in der ersten Kristallisationsstufe gewonnene gereinigte N-Vinylpyrrolidon kann gewünschtenfalls weiteren Kristallisationsstufen unterworfen werden, wie sie beispielsweise in der EP-A 767 169 beschrieben werden. Auf die Offenbarung dieser Schrift wird insoweit Bezug genommen.

Das bei der Kristallisation anfallende gereinigte N-Vinylpyrrolidon enthält in der Regel weniger als 0,7 Gew.-%, insbesondere weniger als 0,1 Gew.-% und besonders bevorzugt weniger als 0,05 Gew.-% Verunreinigungen. Das so gewonnene N-Vinylpyrrolidon genügt den Anforderungen, wie sie im Bereich der Lebensmittelindustrie oder im Pharmabereich gestellt werden.

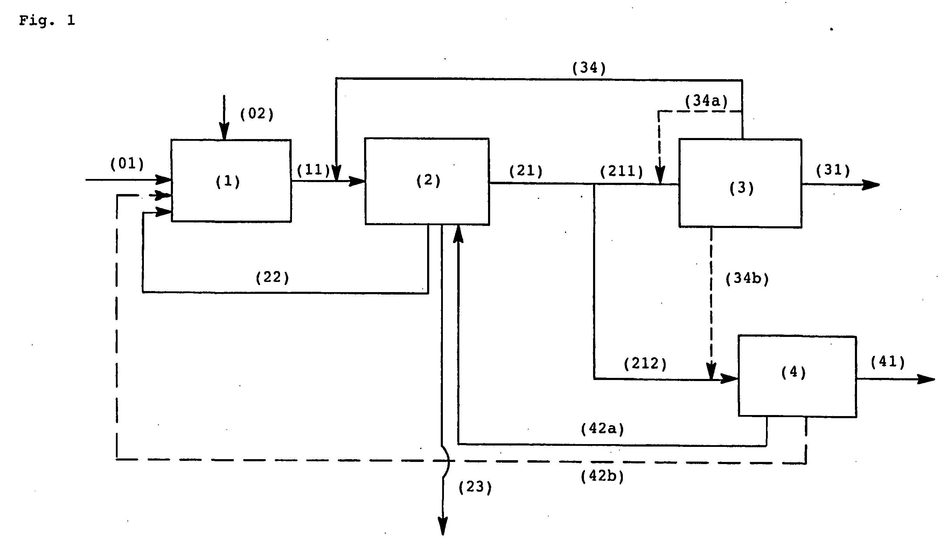

Figur 1 stellt schematisch die Einbindung der erfindungsgemäßen Kristallisation in ein bestehendes kontinuierliches Herstellungsverfahren für N-Vinylpyrrolidon mit destillativer Aufarbeitung dar:

2-Pyrrolidon (Strom 01) und Acetylen (Strom 02) werden in Gegenwart eines Katalysators, z. B. einem Alkalimetallhydroxid, in einem Reaktor (1) unter Druck, z. B. 10 bis 25 bar, bei erhöhter Temperatur, z. B. 145 bis 170 °C, umgesetzt. Der hierbei erhaltene N-Vinylpyrrolidon-haltige Produktstrom wird mittels Destillation (2) in eine 2-Pyrrolidon-haltige Fraktion (22), in eine Schwersiederfraktion (23) und in ein N-Vinylpyrrolidon enthaltendes Rohprodukt (21) aufgetrennt. (22) wird in den Herstellungsprozess (1) rückgeführt. Das N-Vinylpyrrolidon enthaltende Rohprodukt (21) trennt man in einen Teilstrom (211) und einen Teilstrom (212) auf. Teilstrom (211) wird in einem Kristallisator (3) einer Kristallisation unterworfen und in ein durch Kristallisation gereinigtes Produkt (31) und in die Mutterlauge aufgetrennt. Die Mutterlauge wird in einer nicht erfindungsgemäßen Ausführungsform des Verfahrens in den Herstellungsprozess, vorzugsweise vor die Destillation (2) (Strom (34)) und/oder vor die Kristallisation (3) (Strom (34a)) zurückgeführt. Die Mutterlauge kann in einer anderen erfindungsgemäßen Ausführungsform mit dem Teilstrom (212) vereinigt und einer destillativen Aufarbeitung (4) zugeführt werden (Strom (34b)). Hierbei erhält man eine 2-Pyrrolidon-haltige Fraktion, die ganz oder teilweise der Destillation (2) (Strom (42a)) und/oder der Herstellung (1) (Strom (42b)) zugeführt wird, sowie reines N-Vinylpyrrolidon (41). Beide Aufarbeitungsvarianten für die Mutterlauge können parallel durchgeführt werden (z. B. Strom (34) und (34b)).

Mit Hilfe des erfindungsgemäßen Verfahrens kann man in einfacher Weise N-Vinylpyrrolidon in zwei Produktqualitäten erhalten, wobei man die Mengenverhältnisse über die Teilstrommengenverhältnisse T1/T2 in einfacher Weise dem Bedarf anpassen kann. Das bei der Kristallisation anfallende N-Vinylpyrrolidon enthält in der Regel Weniger als 0,1 Gew.-% Verunreinigungen. Durch mehrstufige Kristallisation können Produktqualitäten mit Verunreinigungen von weniger als 100 ppm erhalten werden. Bei der destillativen Aufarbeitung fallen Produktqualitäten mit Verunreinigungen von weniger als 0,5 Gew.-%, insbesondere weniger als 0,1 Gew.-% an.

## Patentansprüche

1. Verfahren zur Gewinnung von reinem N-Vinylpyrrolidon aus N-Vinylpyrrolidon-haltigen Rohprodukten, umfassend ein ein- oder mehrstufiges Kristallisationsverfahren, **dadurch gekennzeichnet, dass** man die Mutterlauge der ersten Kristallisationsstufe einer destillativen Reinigung zuführt.

2. Verfahren nach Anspruch 1, **dadurch gekennzeichnet, dass** man das N-Vinylpyrrolidon-haltige Rohprodukt in zwei Teilströme T1 und T2 aufteilt, wobei man Teilstrom T1 einer Kristallisation und Teilstrom T2 einer destillativen Reinigung zuführt.

3. Verfahren nach Anspruch 2, **dadurch gekennzeichnet, dass** man die Mutterlauge der ersten Kristallisationsstufe dem Teilstrom T2 zuführt.

4. Verfahren nach Anspruch 1, **dadurch gekennzeichnet, dass** man ein N-Vinylpyrrolidon-haltiges Rohprodukt einsetzt, das durch destillative Reinigung (Rohdestillation) des N-Vinylpyrrolidon-haltigen Produktstroms des Herstellungsprozesses erhalten wurde.

5. Verfahren nach einem der vorhergehenden Ansprüche, **dadurch gekennzeichnet, dass** man die Kristallisation einstufig durchführt.

6. Verfahren nach Anspruch 5, **dadurch gekennzeichnet, dass** man die Kristallisation bis zu einem Kristallisationsgrad im Bereich von 5 bis 95 % durchführt.

7. Verfahren nach einem der vorhergehenden Ansprüche, **dadurch gekennzeichnet, dass** man die Kristallisation in Gegenwart von Impfkristalle aus N-Vinylpyrrolidon durchführt.

8. Verfahren nach einem der vorhergehenden Ansprüche, **dadurch gekennzeichnet, dass** man die Kristallisation nach einem dynamischen Kristallisationsverfahren durchführt.

## Claims

1. A process for the isolation of pure N-vinylpyrrolidone from N-vinylpyrrolidone-containing crude products, comprising a single-stage or multistage crystallization process, which comprises passing the mother liquor from the first crystallization stage to a distillative purification.

2. The process according to claim 1, wherein the N-vinylpyrrolidone-containing crude product is divided into two substreams T1 and T2, substream T1 being passed to a crystallization, and substream T2 being passed to distillative purification.

3. The process according to claim 2, wherein the mother liquor from the first crystallization stage is passed to the substream T2.

4. The process according to claim 1, wherein an N-vinylpyrrolidone-containing crude product is used which has been obtained by distillative purification (crude distillation) of the N-vinylpyrrolidone-containing product stream of the preparation process.

5. The process according to any of the preceding claims, wherein the crystallization is carried out in a single stage.

6. The process according to claim 5, wherein the crystallization is carried out up to a degree of crystallization in the range from 5 to 95%.

7. The process according to any of the preceding claims, wherein the crystallization is carried out in the presence of seed crystals of N-vinylpyrrolidone.

8. The process according to any of the preceding claims, wherein the crystallization is carried out by a dynamic crystallization process.

## Revendications

1. Procédé pour la production de N-vinylpyrrolidone pure à partir de produits bruts contenant de la N-vinylpyrrolidone, comprenant un procédé de cristallisation à une ou plusieurs étapes, **caractérisé en ce qu'**on alimente la lessive-mère de la première étape de cristallisation dans une purification par distillation.

2. Procédé selon la revendication 1, **caractérisé en ce qu'**on répartit le produit brut contenant de la N-vinylpyrrolidone en deux flux partiels T1 et T2, où on alimente le flux partiel T1 dans une cristallisation et le flux partiel T2 dans une purification par distillation.

3. Procédé selon la revendication 2, **caractérisé en ce qu'**on alimente la lessive-mère de la première étape de cristallisation dans le flux partiel T2.

4. Procédé selon la revendication 1, **caractérisé en ce qu'**on utilise un produit brut contenant de la N-vinylpyrrolidone, qui a été obtenu par purification par distillation (distillation brute) du flux de produits contenant de la N-vinylpyrrolidone du processus de préparation.

5. Procédé selon l'une quelconque des revendications précédentes, **caractérisé en ce qu'**on réalise la cristallisation en une étape.

6. Procédé selon la revendication 5, **caractérisé en ce qu'**on réalise la cristallisation jusqu'à un degré de cristallisation dans la plage de 5 à 95%.

7. Procédé selon l'une quelconque des revendications précédentes, **caractérisé en ce qu'**on réalise la cristallisation en présence de cristaux d'ensemencement en N-vinylpyrrolidone.

8. Procédé selon l'une quelconque des revendications précédentes, **caractérisé en ce qu'**on réalise la cristallisation selon un procédé de cristallisation dynamique.
